# EUROPEAN PATENT APPLICATION

(11) **EP 4 835 473 A1**
(43) Date of publication of application: **04.11.2026**
(21) Application number: 24912788.7
(22) Date of filing: 24.12.2024
(51) Int. Cl.: G01N 33/53

(54) **METHOD FOR MEASURING IMMUNOSUPPRESSANT IN WHOLE BLOOD**

(30) Priority: 26.12.2023 JP 2023218914
(71) Applicant: Fujirebio Inc., Tokyo 107-0052 (JP)
(72) Inventor: SHIMIZU, Haruka, Tokyo 107-0052 (JP); FUNAKOSHI, Shuichi, Tokyo 107-0052 (JP); ISHII, Yuichi, Tokyo 107-0052 (JP)
(74) Representative: Müller-Boré & Partner Patentanwälte PartG mbB
(86) International application number: PCT/JP2024/045619
(87) International publication number: WO 2025/142901

(57) **Abstract**

The present invention provides a method for measuring an immunosuppressive agent in whole blood by immunoassay, the method which can suppress a non-specific reaction. More specifically, the present invention provides a method for measuring an immunosuppressive agent in whole blood, the method including: (a) mixing whole blood possibly containing an immunosuppressive agent with a treatment liquid to prepare a mixture solution; and (b) measuring a concentration of the immunosuppressive agent in the mixture solution by immunoassay, in which the treatment liquid contains an extractant, and the treatment liquid further contains a nonionic surfactant having a polyoxyethylene alcohol structure at a concentration of 0.5 to 5 mass% and/or has a pH of 4.8 to 6.4.

## Description

### FIELD

The present invention relates to a method for measuring an immunosuppressive agent in whole blood and the like.

### BACKGROUND

Most of immunosuppressive agents such as cyclosporine and tacrolimus are mostly present in an erythrocyte fraction. Therefore, whole blood including an erythrocyte fraction is used as a sample in TDM.

When such an immunosuppressive agent is measured, a pretreatment step for extracting the immunosuppressive agent from the erythrocyte fraction is required. As pretreatment solutions used for extracting an immunosuppressive agent from whole blood, an organic solvent-based pretreatment solution (Patent Literature 1) and a buffer-based pretreatment solution (Patent Literature 2) are known.

In addition, Patent Literature 3 describes treating a blood sample with an acid or an alkali to improve extraction efficiency of an immunosuppressive agent.

### CITATION LIST

### PATENT LITERATURE

Patent Literature 1: JP 2011-508209 A
Patent Literature 2: WO 2009/020468 A
Patent Literature 3: WO 2019/131380 A

### SUMMARY

### TECHNICAL PROBLEM

An organic solvent-based pretreatment solution involves certain considerations such as the need for careful handling. In addition, when measurement is performed with a buffer-based pretreatment agent, a sudden non-specific reaction, which means that non-specific reaction occurs or does not occur for each measurement even in the same specimen, sometimes occurs particularly when a fresh whole blood specimen is measured.

It is the object of the present invention to provide a method for measuring an immunosuppressive agent in whole blood by immunoassay, the method which can prevent a non-specific reaction.

### Solution to Problem

As a result of intensive studies, the present inventors have found that by mixing a specific treatment solution containing a nonionic surfactant having a polyoxyethylene alcohol structure at a concentration of 0.5 to 5 mass% and/or having a pH of 4.8 to 6.4 with whole blood, a non-specific reaction can be suppressed in measurement of the concentration of an immunosuppressive agent in whole blood by immunoassay, and thus have completed the present invention.

That is, the present invention provides [1] to [12] below.
[1] A method for measuring an immunosuppressive agent in whole blood, the method comprising:
   (a) mixing whole blood possibly containing an immunosuppressive agent with a treatment solution to prepare a mixture solution; and
   (b) measuring a concentration of the immunosuppressive agent in the mixture solution by immunoassay,
      wherein the treatment solution contains an extractant, and
      wherein the treatment solution further contains a nonionic surfactant having a polyoxyethylene alcohol structure at a concentration of 0.5 to 5 mass% and/or has a pH of 4.8 to 6.4.
[2] The method according to [1], wherein the nonionic surfactant having the polyoxyethylene alcohol structure is at least one selected from the group consisting of an alcohol ethoxylate and a polyoxyethylene-polyoxypropylene block copolymer.
[3] The method according to [1] or [2], wherein the treatment solution contains the nonionic surfactant having the polyoxyethylene alcohol structure at a concentration of 0.9 to 1.4 mass%.
[4] The method according to any of [1] to [3], wherein the treatment solution further contains a pH buffering agent.
[5] The method according to [4], wherein the pH buffering agent is at least one selected from the group consisting of 2-morpholinoethanesulfonate, phosphate, tartrate, citrate, acetate, trifluoroacetate, phthalate, glycine, and carbonate.
[6] The method according to any of [1] to [5], wherein the immunosuppressive agent is a cyclic polypeptide immunosuppressive agent or a macrolide immunosuppressive agent.
[7] The method according to any of [1] to [6], wherein the immunosuppressive agent is at least one selected from the group consisting of cyclosporine, tacrolimus, everolimus, sirolimus, temsirolimus, zotarolimus, biolimus, novolimus, pimecrolimus, myolimus, myolimus, and deforolimus.
[8] The method according to any of [1] to [7], wherein the extractant contains a chaotropic denaturant and/or a surfactant other than the nonionic surfactant having the polyoxyethylene alcohol structure.
[9] The method according to any of [1] to [8], wherein non-specific reaction in the immunoassay is suppressed when the treatment solution contains the nonionic surfactant having a polyoxyethylene alcohol structure at a concentration of 0.5 to 5 mass% and/or has a pH of 4.8 to 6.4.
[10] The method according to any of [1] to [9], wherein the treatment solution contains the nonionic surfactant having a polyoxyethylene alcohol structure at a concentration of 0.5 to 5 mass% and has a pH of 4.8 to 6.4.
[11] A kit for measuring an immunosuppressive agent in whole blood by immunoassay, the kit comprising a treatment solution,
   wherein the treatment solution contains an extractant, and
   wherein the treatment solution further contains a nonionic surfactant having a polyoxyethylene alcohol structure at a concentration of 0.5 to 5 mass% and/or has a pH of 4.8 to 6.4.
[12] A method for suppressing a non-specific reaction in immunoassay for measuring an immunosuppressive agent in whole blood, the method comprising:
   (a) mixing whole blood possibly containing an immunosuppressive agent with a treatment solution to prepare a mixture solution,
      wherein the treatment solution contains an extractant, and
      wherein the treatment solution further contains a nonionic surfactant having a polyoxyethylene alcohol structure at a concentration of 0.5 to 5 mass% and/or has a pH of 4.8 to 6.4.

### EFFECTS OF INVENTION

The present invention can suppress a non-specific reaction such as a sudden non-specific reaction when measuring the concentration of an immunosuppressive agent in whole blood by immunoassay.

### DESCRIPTION OF EMBODIMENTS

### [1. Method for measuring immunosuppressive agent in whole blood]

The present invention provides a method for measuring an immunosuppressive agent in whole blood by immunoassay, the method including:
(a) mixing whole blood possibly containing an immunosuppressive agent with a treatment liquid to prepare a mixture solution; and
(b) measuring the concentration of the immunosuppressive agent in the mixture solution by immunoassay.

### [1-1. Step (a)]

### [Whole Blood]

In the present invention, the blood sample that is a specimen is whole blood, which possibly contains an immunosuppressive agent. Such whole blood can be collected from an animal to which an immunosuppressive agent has been administered. The animal from which such whole blood is derived is preferably a mammal (e.g., primates such as human, monkey, chimpanzee; rodents such as mice, rats, and rabbits; livestock and working animals such as cattle, pigs, horses, goats, and sheep), more preferably a primate, and particularly preferably a human. Immunosuppressive agents are mostly present in the blood fraction (erythrocyte fraction) in whole blood. The whole blood may be further collected from a mammal suffering from a disease requiring immune suppression (e.g., autoimmune disease, rheumatoid arthritis, myasthenia gravis, Crohn's disease, lupus nephritis, active ulcerative colitis, polymyositis, graft-versus-host disease (GVDH), interstitial pneumonia complicating dermatomyositis) or a mammal having undergone transplantation of an organ, a tissue or a cell (e.g., heart transplant, kidney transplant, bone marrow transplant, lung transplant, liver transplant, pancreas transplant, small intestine transplant). Whole blood collected from such mammals may be subjected to a pretreatment (e.g., filtration, heating, or hemolysis).

The whole blood may also preferably be fresh whole blood. Fresh whole blood refers to a specimen within 7 days after collection, preferably within 3 days, and more preferably within 1 day. The storage temperature after blood collection is usually 2 to 30°C, and preferably 2 to 10°C.

In addition, the whole blood may be a specimen that has been frozen (frozen specimen) or may be a specimen that has not been frozen (non-frozen specimen), and may be preferably a non-frozen specimen.

The frozen specimen is a specimen in which whole blood is frozen and then thawed. The freezing temperature and the freezing time are not particularly limited as long as the specimen is frozen, and the freezing temperature can be, for example, -100 to -10°C, and is preferably -90 to - 50°C. The freezing time is, for example, 30 minutes or more, 1 hour or more, or 1.5 hours or more. The melting temperature and the melting time are not particularly limited as long as the frozen specimen is melted, and the melting temperature is, for example, room temperature (e.g., 1 to 30°C or 15 to 25°C).

### [Treatment Liquid]

The treatment solution contains an extractant, and further contains a nonionic surfactant having a polyoxyethylene alcohol structure at a concentration of 0.5 to 5 mass%, and/or has a pH of 4.8 to 6.4. The treatment solution may further contain a pH buffering agent described later.

### -Extractant-

In the present invention, the extractant is a substance which can lyse cells, dissociate an immunosuppressive agent in whole blood from its bound protein, and extract the immunosuppressive agent. In the present invention, the extractant does not comprise a nonionic surfactant having a polyoxyethylene alcohol structure described later.

The extractant preferably comprises a chaotropic denaturant and/or a surfactant other than the nonionic surfactant having a polyoxyethylene alcohol structure.

Examples of the chaotropic denaturant include urea, thiourea, guanidine hydrochloride, guanidine thiocyanate, sodium salicylate, sodium thiocyanate, sodium perchlorate, acetamide, and formamide, and urea is preferable.

As the chaotropic denaturant, one of the above-described components may be used alone, or a mixture of two or more types (e.g., two, three, four, or five types) may be used.

The concentration of the extractant in the treatment solution is usually 3 mass% or more, preferably 6 mass% or more, and more preferably 12 mass% or more. The upper limit thereof is usually 63 mass% or less, preferably 46 mass% or less, and more preferably 37 mass% or less. Therefore, the concentration of the extractant in the treatment solution is usually 3 to 63 mass%, preferably 6 to 46 mass%, and more preferably 12 to 37 mass%.

The concentration of the extractant in the mixture solution of the treatment solution and the whole blood (hereinafter, simply referred to as a "mixture solution") is usually 2.5 mass% or more, preferably 5 mass% or more, and more preferably 10 mass% or more. The upper limit thereof is usually 52.5 mass% or less, preferably 38.4 mass% or less, and more preferably 30.9 mass% or less. Therefore, the concentration of the extractant in the mixture solution is usually 2.5 to 52.5 mass%, preferably 5 to 38.4 mass%, and more preferably 10 to 30.9 mass%.

When the treatment solution contains a chaotropic denaturant, the concentration in the treatment solution is usually 0.5 M (3 mass%) or more, preferably 1 M (6 mass%) or more, and more preferably 2 M (12 mass%) or more. The upper limit thereof is usually 8 M (48 mass%) or less, preferably 6 M (36 mass%) or less, and more preferably 5 M (30 mass%) or less. Therefore, the concentration of the chaotropic denaturant in the treatment solution is usually 0.5 to 8 M (3 to 48 mass%), preferably 1 to 6 M (6 to 36 mass%), and more preferably 2 to 5 M (12 to 30 mass%). When the concentration of the chaotropic denaturant satisfies the above numerical range, the effect of the present invention can be more suitably exhibited.

When the treatment solution contains the chaotropic denaturant, the concentration in the mixture solution is usually 0.4 M (2.5 mass%) or more, preferably 0.8 M (5 mass%) or more, and more preferably 1.6 M (10 mass%) or more. The upper limit thereof is usually 6.7 M (40 mass%) or less, preferably 5 M (30 mass%) or less, and more preferably 4.2 M (25 mass%) or less. Therefore, the concentration of the chaotropic denaturant in the mixture solution is usually 0.4 to 6.7 M (2.5 to 40 mass%), preferably 0.8 to 5 M (5 to 30 mass%), and more preferably 1.6 to 4.2 M (10 to 25 mass%). When the concentration of the chaotropic denaturant satisfies the above numerical range, the effect of the present invention can be more suitably exhibited.

Examples of the surfactant other than the nonionic surfactant having a polyoxyethylene alcohol structure include a nonionic surfactant other than the nonionic surfactant having a polyoxyethylene alcohol structure, a cationic surfactant, an anionic surfactant, and an amphoteric surfactant.

The nonionic surfactant other than the nonionic surfactant having a polyoxyethylene alcohol structure may be a nonionic surfactant other than the components listed in the section "Nonionic surfactant having polyoxyethylene alcohol structure" described later, and examples thereof include N-D-gluco-N-methylalkanamide (for example, MEGA series (e.g., octanoyl-N-methylglucamide (MEGA 8) and decanoyl-N-methylglucamide (MEGA 10)).

Examples of the cationic surfactant include quaternary ammonium salts. Examples of the salts include salts of halogen (e.g., fluorine, chlorine, bromine, and iodine). Examples of the quaternary ammonium salts include Cn alkyltrimethylammonium bromide (CnTAB) and Cn alkyltrimethylammonium chloride (CnTAC). Examples of the CnTAB include octyltrimethylammonium bromide (C8TAB), nonyltrimethylammonium bromide (C9TAB), dodecyltrimethylammonium bromide (C12TAB), tetradecyltrimethylammonium bromide (C14TAB), and hexadecyltrimethylammonium bromide (C16TAB). Examples of the CnTAC include dodecyltrimethylammonium chloride (C12TAC), tetradecyltrimethylammonium chloride (C14TAC), hexadecyltrimethylammonium chloride (C16TAC), and octadecyltrimethylammonium chloride (C18TAC).

Examples of the anionic surfactant include carboxylic acid type surfactants (e.g., N-decanoylsarcosine sodium (NDS), and N-lauroylsarcosine sodium hydrate (NLS)), sulfonic acid type surfactants (e.g., sodium 1-nonansulfonate (NSS), and sodium dodecylbenzenesulfonate (SDBS)), carboxylic acid type-sulfonic acid type surfactants (e.g., sodium chondroitin sulfate (CSSS)), and sulfate ester type surfactants (e.g., sodium dodecyl sulfate (SDS)).

Examples of the amphoteric surfactant include quaternary ammonium-sulfonic acid type surfactants. Examples of the quaternary ammonium-sulfonic acid type surfactants include 3-[(3-cholamidopropyl) dimethylammonio]-1-propane sulfonate (CHAPS), 3-[(3-cholamidopropyl) dimethylammonio]-2-hydroxy-1-propane sulfonate (CHAPSO), 3-(N,N-dimethyloctylammonio) propane sulfonate (C8APS), 3-(decyldimethylammonio) propane sulfonate (C10APS), N-dodecyl-N,N-dimethyl-3-ammonio-1-propane sulfonate (C12APS), 3-(N,N-dimethylmyristylammonio) propane sulfonate (C14APS), 3-(N,N-dimethylpalmitylammonio) propane sulfonate (C16APS), dimethylethylammonium propane sulfonate (NDSB-195), 3-[dimethyl-(2-hydroxyethyl) ammonio]-1-propane sulfonate (NDSB-211), and 3-(benzenedimethylammonio) propane sulfonate (NDSB-256).

The surfactant other than the nonionic surfactant having a polyoxyethylene alcohol structure is preferably a nonionic surfactant other than nonionic surfactant having a polyoxyethylene alcohol structure or an amphoteric surfactant, more preferably N-D-gluco-N-methylalkanamide or a quaternary ammonium-sulfonic acid type surfactant, and still more preferably octanoyl-N-methylglucamide (MEGA8) or 3-[(3-cholamidopropyl) dimethylammonio]-1-propane sulfonate (CHAPS).

As the surfactant other than the nonionic surfactant having a polyoxyethylene alcohol structure, one of the above-described components may be used alone, or a mixture of two or more (e.g., two, three, four, or five types) may be used in combination.

When the treatment solution contains a surfactant other than the nonionic surfactant having a polyoxyethylene alcohol structure, the concentration of the surfactant in the treatment solution is usually 0.01 mass% or more, preferably 0.1 mass% or more, and more preferably 1 mass% or more. The upper limit thereof is usually 15 mass% or less, preferably 10 mass% or less, and more preferably 7 mass% or less. Therefore, the concentration of the surfactant other than the nonionic surfactant having a polyoxyethylene alcohol structure in the treatment solution is usually 0.01 to 15 mass%, preferably 0.1 to 10 mass%, and more preferably 1 to 7 mass%. When the concentration of the surfactant other than the nonionic surfactant having a polyoxyethylene alcohol structure in the treatment solution satisfies the above numerical range, the effect of the present invention can be more suitably exhibited.

When the treatment solution contains the surfactant other than the nonionic surfactant having a polyoxyethylene alcohol structure, the concentration in the mixture solution is usually 0.008 mass% or more, preferably 0.08 mass% or more, and more preferably 0.8 mass% or more. The upper limit thereof is usually 12.5 mass% or less, preferably 8.4 mass% or less, and more preferably 5.9 mass% or less. Therefore, the concentration of the surfactant other than the nonionic surfactant having a polyoxyethylene alcohol structure in the mixture solution is usually 0.008 to 12.5 mass%, preferably 0.08 to 8.4 mass%, and more preferably 0.8 to 5.9 mass%. When the concentration of the surfactant other than the nonionic surfactant having a polyoxyethylene alcohol structure in the mixture solution satisfies the above numerical range, the effect of the present invention can be more suitably exhibited.

### -Nonionic surfactant having polyoxyethylene alcohol structure-

The nonionic surfactant having a polyoxyethylene alcohol structure is preferably a linear or branched chain compound (compound not containing a cyclic structure) containing a structure represented by -O-(-CH₂-CH₂-O-)ₓ-H (polyoxyethylene alcohol structure). x may be an integer of 1 or more, preferably an integer of 5 or more, more preferably an integer of 15 or more, and even more preferably an integer of 20 or more. As well, x may be an integer of 400 or less, preferably an integer of 350 or less, more preferably an integer of 300 or less, even more preferably an integer of 280 or less, and particularly preferably an integer of 270 or less. More specifically, x may be an integer of from 1 to 400, preferably an integer of from 5 to 350, more preferably an integer of from 15 to 300, even more preferably an integer of from 20 to 280, and particularly preferably an integer of from 20 to 270. Herein, the value of x represents an average value.

The nonionic surfactant having a polyoxyethylene alcohol structure may be formed by only single bonds or may contain an unsaturated bond(s) (double/triple bond(s)), and is preferably a compound formed by only single bonds. Such a compound may include, for example, alkyl structures (linear or branched chain), and/or polyoxyalkylene structures (linear or branched chain) as a structure(s) other than the polyoxyethylene alcohol structure. The alkyl structure may be a C₆₋₃₁ alkyl structure, for example, and is preferably a C₆₋₃₁ linear alkyl structure. The alkylene in the polyoxyalkylene structure may be a C₁₋₆ alkylene, for example. Examples of the C₁₋₆ alkylene include C₁ alkylene (methylene), C₂ alkylenes (ethylene group and ethylidene group), C₃ alkylenes (propylidene, propylene, trimethylene and isopropylidene), C₄ alkylenes (e.g., tetramethylene), C₅ alkylenes (e.g., pentamethylene), and C₆ alkylenes (e.g., hexamethylene). Such a compound may be composed of a carbon atom(s), a hydrogen atom(s), and an oxygen atom(s).

More specifically, examples of such a nonionic surfactant having a polyoxyethylene alcohol structure include alcohol ethoxylates and polyoxyethylene-polyoxyalkylene block copolymers (e.g., polyoxyethylene-polyoxypropylene block copolymers).

The alcohol ethoxylate is also referred to as a poly(oxyethylene) alkyl ether, and is a compound in which a hydrophilic polyoxyethylene (POE) chain(s) is linked to a hydrophobic alkyl group(s) via an ether bond(s). The alcohol ethoxylate can be represented by the following formula (1). 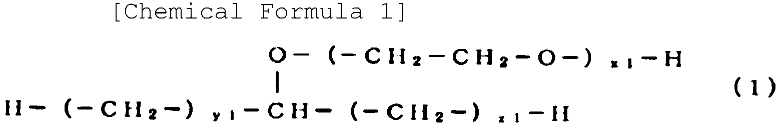
wherein x1 is an integer of 1 or more;
y1 is an integer of 0 or more;
z1 is an integer of 0 or more; and
y1 ≥ z1.

Herein, the values of x1, y1, and z1 represent average values.

x1 may be an integer of 1 or more, preferably an integer of 5 or more, more preferably an integer of 10 or more, even more preferably an integer of 15 or more, and particularly preferably an integer of 20 or more. As well, x1 may be an integer of 300 or less, preferably an integer of 250 or less, more preferably an integer of 200 or less, even more preferably an integer of 150 or less, and particularly preferably an integer of 100 or less, 80 or less, 60 or less, or 50 or less. More specifically, x1 may be an integer of from 1 to 300, preferably an integer of from 5 to 250, more preferably an integer of from 10 to 200, even more preferably an integer of from 15 to 150, and particularly preferably an integer of from 20 to 100, from 20 to 80, from 20 to 60, or from 20 to 50.

y1 is an integer of 0 or more, preferably an integer of 1 or more, and more preferably an integer of 5 or more. y1 may also be an integer of 30 or less, preferably an integer of 20 or less, and still more preferably an integer of 13 or less. More specifically, y1 may be an integer of from 0 to 30, preferably an integer of from 1 to 20, and even more preferably an integer of from 5 to 15.

z1 is an integer of 0 or more, and may be an integer of 1 or more, and is preferably 0. z1 is also an integer of 15 or less, preferably an integer of 10 or less, and may be an integer of 7 or less. More specifically, z1 is an integer of from 0 to 15, preferably an integer of from 1 to 10, still more preferably an integer of from 0 to 7, and further preferably 0.

Preferably, y1 and z1 may satisfy a relation of y1 ≥ z1. y1 and z1 may also satisfy a relation that y1 + z1 is an integer of from 5 to 30. Preferably, y1 + z1 may satisfy a relation that y1 + z1 is an integer of from 10 to 14.

The alcohol ethoxylate may be either a branched chain alcohol ethoxylate or a linear alcohol ethoxylate. Preferably, the alcohol ethoxylate may be a linear alcohol ethoxylate.

The branched chain alcohol ethoxylate corresponds to a compound represented by the above formula (1) in which y1 is an integer of 1 or more and z1 is an integer of 1 or more. Examples of such a branched-chain alcohol ethoxylate include polyoxyethylene (40) sec-tridecyl ether. Specific examples of such a branched-chain alcohol ethoxylate include TERGITOL (registered trademark) series compounds such as Tergitol 15-S-40.

The linear alcohol ethoxylate corresponds to a compound represented by the above formula (1) in which z1 is 0. Specific examples of the linear alcohol ethoxylate include BRIJ (registered trademark) series compounds such as Brij 58 and Brij S100. The linear alcohol ethoxylate can be represented also by the following formula (1').
[Chemical Formula 2]

H-(-CH₂-)y₁-O-(-CH₂-CH₂-O-)ₓ₁-H (1')

wherein x1 is an integer of 1 or more; and
y1 is an integer of 1 or more.

The preferred examples of x1 and y1 in formula (1') are the same as the preferred examples of x1 and y1 in formula (1), respectively.

Preferably, the compound represented by formula (1) is as follows:
x1 is an integer of from 40 to 100;
y1 is an integer of 0 or more;
z1 is an integer of 0 or more;
y1 ≥ z1; and
y1 + z1 is an integer of from 5 to 30.
(Preferred ranges of x1, y1, and z1 are the same as the described above.)

More specific examples of the alcohol ethoxylate corresponding to a compound represented by formula (1) include Brij 58, Tergitol 15-S-40, and Brij S100. Preferably, the alcohol ethoxylate is Brij 58.

The "polyoxyethylene-polyoxyalkylene block copolymer" is a block copolymer that contains a polyoxyethylene block and a polyoxyalkylene block. The alkylene in the polyoxyalkylene block may be C₁₋₆ alkylene, for example. Examples of the C₁₋₆ alkylene include C₁ alkylene (methylene), C₂ alkylenes (ethylene and ethylidene), C₃ alkylenes (propylidene, propylene, trimethylene and isopropylidene), C₄ alkylenes (e.g., tetramethylene), C₅ alkylenes (e.g., pentamethylene), and C₆ alkylenes (e.g., hexamethylene). Examples of the polyoxyethylene-polyoxyalkylene block copolymer include a block copolymer having a structure of HO-[polyoxyethylene block]-[polyoxyalkylene block]-[polyoxyethylene block]-OH. The polyoxyethylene-polyoxyalkylene block copolymer may be preferably a polyoxyethylene-polyoxypropylene block copolymer.

The "polyoxyethylene-polyoxypropylene block copolymer" is a compound represented by the following formula (2):
[Chemical Formula 3]

HO-(-CH₂-CH₂-O-)ₓ₂-((-CH(-CH₃))CH₂-O-)_{y2}-(-CH₂-CH₂-O-)ₓ₂-H (2)

wherein each of x2, y2, and z2 is an integer of 1 or more.

Herein, the values of x2, y2, and z2 represent average values.

Each of x2 and z2 is an integer of 1 or more. The sum of x2 and z2 may be an integer of 2 or more, preferably an integer of 20 or more, more preferably an integer of 80 or more, and particularly preferably an integer of 150 or more. As well, the sum of x2 and z2 is an integer of 400 or less, preferably an integer of 350 or less, more preferably an integer of 300 or less, and particularly preferably an integer of 270 or less. More specifically, the sum of x2 and z2 may be an integer of from 2 to 400, preferably an integer of from 20 to 350, more preferably an integer of from 80 to 300, and particularly preferably an integer of from 150 to 270.

y2 may be an integer of 1 or more, preferably an integer of 5 or more, more preferably an integer of 10 or more, even more preferably an integer of 15 or more, and particularly preferably an integer of 20 or more. As well, y2 is an integer of 200 or less, preferably an integer of 150 or less, more preferably an integer of 100 or less, even more preferably an integer of 80 or less, and particularly preferably an integer of 70 or less. More specifically, x1 may be an integer of from 1 to 200, preferably an integer of from 5 to 150, more preferably an integer of from 10 to 100, even more preferably an integer of from 15 to 80, and particularly preferably an integer of from 20 to 70.

Examples of the polyoxyethylene-polyoxypropylene block copolymer include poloxamer 188, poloxamer 388 and poloxamer 407. Specific examples of the polyoxyethylene-polyoxypropylene block copolymer include PLURONIC (registered trademark) series compounds such as Pluronic F68, Pluronic F108 and Pluronic F127.

Preferably, the compound represented by formula (2) is as follows:
x2 is an integer of 1 or more;
y2 is an integer of from 15 to 80;
z2 is an integer of 1 or more; and
x2 + z2 is an integer of from 80 to 280.
(Preferred ranges of x2, y2, and z2 are same as the described above.)

More specifically, examples of the polyoxyethylene-polyoxypropylene block copolymer corresponding to the compound represented by formula (2) include poloxamer 188 (e.g., Pluronic F68), poloxamer 108 (e.g., Pluronic F38), poloxamer 217 (e.g., Pluronic F77), poloxamer 237 (e.g., Pluronic F87), poloxamer 238 (e.g., Pluronic F88), poloxamer 288 (e.g., Pluronic F98), poloxamer 388 (e.g., Pluronic F108), and poloxamer 407 (e.g., Pluronic F127). Preferably, examples of the polyoxyethylene-polyoxypropylene block copolymer include poloxamer 188 (e.g., Pluronic F68) and poloxamer 388 (e.g., Pluronic F108).

For the reference, the relationship between the nonionic surfactant (alcohol ethoxylate/polyoxyethylene-polyoxypropylene block copolymer) and the compound represented by the above formula (1) or (2) is as follows.

**[Table A]**

| Table A. Relationship between alcohol ethoxylate and compound represented by the above formula (1) | | |
|---|---|---|
| Nonionic surfactant | x1 | y1 |
| Briji 58 | 20 | 16 |

**[Table B]**

| Table B. Relationship between polyoxyethylene-polypropylene block copolymer and compound represented by the above formula (2) | | |
|---|---|---|
| Nonionic surfactant | x2 + z2 | y2 |
| Pluronic F68 | 153 | 29 |
| Pluronic F108 | 265 | 50 |

The average molecular weight of the nonionic surfactant having a polyoxyethylene alcohol structure is preferably 900 or more, more preferably 1000 or more, and still more preferably 1100 or more. The upper limit thereof is preferably 18000 or less, more preferably 15000 or less, and still more preferably 14800 or less. Therefore, the average molecular weight of the nonionic surfactant having a polyoxyethylene alcohol structure is preferably 900 to 18000, more preferably 1000 to 15000, and still more preferably 1100 to 14800. When the average molecular weight satisfies the above numerical range, the effect of the present invention can be more suitably exhibited. The "average molecular weight" means a weight average molecular weight unless otherwise specified.

The nonionic surfactant having a polyoxyethylene alcohol structure is preferably an alcohol ethoxylate or a polyoxyethylene-polyoxypropylene block copolymer, and more preferably polyoxyethylene (20) cetyl ether, poloxamer 188, or poloxamer 388.

Specific examples of such a nonionic surfactant include PLURONIC (registered trademark) series compounds such as Pluronic F-68 and Pluronic F-108, and BRIJ (registered trademark) series compounds such as Brij 58.

As the nonionic surfactant having a polyoxyethylene alcohol structure, one type of nonionic surfactant may be used, or a plurality of types (e.g., two, three, or four types) of nonionic surfactants may be used in combination.

The concentration of the nonionic surfactant having a polyoxyethylene alcohol structure in the treatment solution is preferably 0.5 mass% or more, more preferably 0.8 mass% or more, still more preferably 0.9 mass% or more, and even more preferably 1.0 mass% or more (and may be more than 1.0 mass%, as necessary). This helps suppress a non-specific reaction in the immunoassay. The upper limit is preferably 5.0 mass% or less, more preferably 4.0 mass% or less, still more preferably 3.0 mass% or less, further preferably 2.0 mass% or less, and most preferably 1.4 mass% or less. This helps prevent a decrease in detection sensitivity in the immunoassay. Therefore, the concentration of the nonionic surfactant having a polyoxyethylene alcohol structure in the treatment solution is preferably 0.5 to 5.0 mass%, more preferably 0.8 to 4.0 mass%, still more preferably 0.9 to 3.0 mass%, even more preferably 1.0 to 2.0 mass%, and even more preferably 1.0 to 1.4 mass%.

The concentration of the nonionic surfactant having a polyoxyethylene alcohol structure in the mixture solution is preferably 0.4 mass% or more, more preferably 0.6 mass% or more, still more preferably 0.75 mass% or more, even more preferably 0.8 mass% or more, and most preferably 0.9 mass% or more. This helps suppress a non-specific reaction in the immunoassay. The upper limit is preferably 4.2 mass% or less, more preferably 3.4 mass% or less, still more preferably 2.5 mass% or less, even more preferably 1.7 mass% or less, and most preferably 1.2 mass% or less. This helps prevent a decrease in detection sensitivity in the immunoassay. Therefore, the concentration of the nonionic surfactant having a polyoxyethylene alcohol structure in the mixture solution is preferably 0.4 to 4.2 mass%, more preferably 0.6 to 3.4 mass%, still more preferably 0.75 to 3.4 mass%, even more preferably 0.8 to 2.5 mass%, even more preferably 0.8 to 1.7 mass%, and most preferably 0.9 to 1.2 mass%.

### -pH-

The pH of the treatment solution is preferably 4.8 or more, more preferably 5.5 or more, still more preferably 6.0 or more, and most preferably 6.1 or more. When the lower limit of the pH satisfies the above numerical range, a non-specific reaction can be suppressed. The upper limit is preferably 6.4 or less, more preferably 6.3 or less, and most preferably 6.2 or less. When the upper limit of the pH satisfies the above numerical range, a decrease in sensitivity in the immunoassay can be prevented. Therefore, the pH of the treatment solution is preferably 4.8 to 6.4, more preferably 5.5 to 6.3, still more preferably 6.0 to 6.3, and most preferably 6.1 to 6.2.

The pH can be measured using a method known in the art. Preferably, it is possible to employ a value measured at 25°C with a pH meter having a glass electrode as the pH.

### -pH Buffering agent-

The pH of the treatment solution can be adjusted with a pH buffering agent. That is, the treatment solution may further contain a pH buffering agent. Examples of the pH buffering agent include 2-morpholinoethanesulfonate (MES), phosphate, tartrate, citrate, acetate, trifluoroacetate, phthalate, glycine, and carbonate. Among them, 2-morpholinoethanesulfonate is preferable.

### [Immunosuppressive agent]

The immunosuppressive agent is not particularly limited as long as it is a compound having an action of reducing or inhibiting the activity of the immune system, and a cyclic polypeptide immunosuppressive agent or a macrolide immunosuppressive agent is preferable. The macrolide immunosuppressive agent includes a macrolide structure (cyclic lactone structure composed of 12 or more atoms).

Examples of the cyclic polypeptide immunosuppressive agent include cyclosporine (cyclosporine A). Examples of the macrolide immunosuppressive agent include tacrolimus (FK506), sirolimus (rapamycin), and derivatives thereof (e.g., everolimus and temsirolimus), zotarolimus, biolimus, novolimus, pimecrolimus, myolimus, myolimus, and deforolimus.

Among the above immunosuppressive agents, cyclosporine, tacrolimus, sirolimus, everolimus, temsirolimus, zotarolimus, biolimus, novolimus, pimecrolimus, myolimus, myolimus, and deforolimus are preferable, and cyclosporine is more preferable.

### -Mixing treatment-

In step (a), the above-described whole blood is mixed with the above-described treatment solution. This enables the immunosuppressive agent that may be present in whole blood to be extracted from the whole blood (erythrocyte fraction). The mixing can be performed in any manner. For example, the mixing can be performed by inversion mixing and/or stirring, preferably stirring. The order of the mixing of whole blood and the treatment solution is not particularly limited. The mixing time is not particularly limited as long as it is sufficient time for mixing whole blood with the treatment solution, and may be, for example, 1 second to 5 minutes, or 10 seconds to 1 minute. The temperature during the mixing may be, for example, 4 to 37°C, or 15 to 30°C. The whole blood and the treatment solution may be mixed and then incubated. The incubation time may be 1 second to 60 minutes, for example, preferably 5 seconds to 20 minutes. The temperature at the time of incubation is not particularly limited, and is 20 to 45°C, preferably 30 to 39°C.

### [1-2. Step (b)]

### [Immunoassay]

In the immunoassay, the concentration of the immunosuppressive agent in the mixture solution is measured using an antibody.

Examples of the immunoassay include enzyme immunoassay (EIA) (e.g., a chemiluminescent EIA (CLEIA) and an enzyme-linked immuno sorbent assay (ELISA)), fluorescence immunoassay, chemiluminescent immunoassay, electrochemiluminescent immunoassay, agglutination method, immunostaining, flowmetry method, biolayer interferometry, in situ PLA method, chemical amplification type luminescence proximity homogeneous assay, line blotting, and Western blotting. Among them, a sandwich immunoassay method (enzyme-linked immunosorbent assay (ELISA)) is preferable.

The antibody used in the present invention is not particularly limited as long as it is an antibody against an immunosuppressive agent to be measured, and is preferably an antibody against a cyclic polypeptide immunosuppressive agent or a macrolide immunosuppressive agent. Such an antibody may be either a polyclonal antibody or a monoclonal antibody, and is preferably a monoclonal antibody. The antibody may be of any isotype of an immunoglobulin (e.g., IgG, IgM, IgA, IgD, IgE, IgY, or HCAb). The antibody may also be a full-length antibody. The full-length antibody refers to an antibody (e.g., an antibody comprising two Fab moieties and Fc moieties) containing a heavy chain and a light chain each containing a variable region and a constant region. The antibody may also be an antibody fragment derived from such a full-length antibody. The antibody fragment is a part of a full-length antibody, and examples thereof include a constant region deletion antibody (e.g., F(ab')₂, Fab', Fab, Fv, or VHH). The antibody may also be a modified antibody such as a single chain antibody. Preferably, the antibody may be an IgG, an IgM, or an antibody fragment thereof. In the present invention, not only one type of antibody but also two or more types of antibodies (e.g., two or three types) can be used.

An antibody to the target immunosuppressive agent can be produced using any method known in the art. For example, an antibody can be produced using a target immunosuppressive agent as an antigen. When an antibody against the target immunosuppressive agent is commercially available, such a commercially available product may also be used.

One or more types of antibodies (e.g., one or two types) against the target immunosuppressive agent can be used in the present invention. A secondary antibody against the antibody can also be used in the present invention.

The antibody may be immobilized on a solid phase. Herein, the antibody immobilized on a solid phase may be simply referred to as a solid-phase antibody. Examples of the solid phase include a solid phase (containers such as well plates, microchannels, glass capillaries, nanopillars, monolithic columns, tubes; and a support such as a plate, a membrane, or a filter paper.) which can store or mount a liquid phase, and a solid phase (e.g., solid phase carriers such as particles) which can be suspended or disperse in a liquid phase. Examples of the material of the solid phase include glass, plastic, metal, and carbon. As the material of the solid phase, a nonmagnetic material or a magnetic material can be used, and the solid phase carrier is preferably a magnetic material from the viewpoint of convenience of operation and the like. As a method for immobilizing an antibody on a solid phase, any method known in the art can be used. Examples of such a method include a physical adsorption method, a covalent binding method, a method using an affinity substance (e.g., biotin or streptavidin), and an ionic binding method.

The antibody may be labeled with a labeling substance. Herein, the antibody labeled with a labeling substance may be simply referred to as a labeled antibody. Examples of the labeling substance include an enzyme (e.g., peroxidase, alkaline phosphatase, luciferase, β-galactosidase), an affinity substance (e.g., streptavidin, biotin), a fluorescent substance or a protein (e.g., fluorescein, fluorescein isothiocyanate, rhodamine, green fluorescent protein, red fluorescent protein), a luminescent or light-absorbing substance (e.g., Luciferin, Aequorin, or Acridinium), and a radioactive substance (e.g., ³H, ¹⁴C, ³²P, ³⁵S, or ¹²⁵I).

Steps (a) and (b) may be performed in parallel or separately. For example, when an antibody against the above-described immunosuppressive agent is used in mixing whole blood and the treatment solution, extraction of the immunosuppressive agent from the erythrocyte fraction by the treatment solution and measurement of the concentration of the immunosuppressive agent by immunoassay using the antibody can be performed in parallel. For example, whole blood, a treatment solution, and an antibody against the above-described immunosuppressive agent may be mixed at the same time. As the antibody, a second antibody against an immunosuppressive agent (e.g., labeled antibody) different from the first antibody against an immunosuppressive agent (e.g., solid-phase antibody) may be further used. On the other hand, for example, when whole blood, a treatment solution, and an antibody against the above-described immunosuppressive agent are not mixed at the same time, extraction of the immunosuppressive agent by the treatment solution and measurement of the concentration of the immunosuppressive agent can be separately performed. In this case, steps (a) and (b) can be carried out continuously or discontinuously. When steps (a) and (b) are performed discontinuously, step (a) is usually performed, and then step (b) is performed. In the step (a), the whole blood mixed with the treatment solution may be subjected to further treatment. Examples of such a treatment include a preliminary treatment (e.g., filtration, heating, or hemolysis), a change in pH, and a treatment with a predetermined chemical substance.

In certain embodiments, the method of the invention may comprise the following steps:
(a) mixing whole blood containing an immunosuppressive agent with a treatment solution to prepare a mixture solution; and
(b) measuring the concentration of the immunosuppressive agent in the mixture solution by immunoassay.

Such a method is useful for measuring counts of immunosuppressive agents for whole blood containing immunosuppressive agents in a method for measuring immunosuppressive agents in whole blood.

In another certain embodiment, the method of the invention may comprise the following steps:
(a) mixing whole blood not containing an immunosuppressive agent with a treatment solution to prepare a mixture solution; and
(b) measuring the concentration of the immunosuppressive agent in the mixture solution by immunoassay.

Such a method is useful for measuring background counts for whole blood not containing an immunosuppressive agent in a method for measuring an immunosuppressive agent in whole blood.

### [2. Kit for measuring immunosuppressive agent]

The present invention also provides a kit for measuring an immunosuppressive agent in whole blood by immunoassay, including a treatment solution, which contains an extractant and further contains a nonionic surfactant having a polyoxyethylene alcohol structure at a concentration of 0.1 to 5 mass% and/or has a pH of 4.8 to 6.4.

The conditions of the pH of the nonionic surfactant having a polyoxyethylene alcohol structure, the extractant, and the treatment solution contained in the treatment solution are as described above.

The kit of the present invention may also contain a pH buffering agent. The pH buffering agent is as described above.

The kit of the present invention may further contain an antibody against an immunosuppressive agent. The antibody against the immunosuppressive agent is as described above.

The kit of the present invention may also contain an immunosuppressive agent (preparation) to be measured. The immunosuppressive agent can be used, for example, as a positive control and/or for preparing a calibration curve for quantification.

The reagent of the present invention may further contain components necessary for immunoassay. Examples of such components include labeling substances and enzymes as described above, substrates thereof, diluents, stabilizers for secondary antibodies and antibodies, and instruments which can collect samples from mammals (e.g., syringes and biopsy needles).

### [3. Method for suppressing non-specific reaction in immunoassay for measuring immunosuppressive agent in whole blood]

The present invention also provides a method for suppressing a non-specific reaction in an immunoassay for measuring an immunosuppressive agent in whole blood, the method including the following steps.
(a) mixing whole blood possibly containing an immunosuppressive agent with a treatment solution to prepare a mixture solution.

The above method can be performed in the same manner as the step (a) in the method for measuring an immunosuppressive agent in whole blood. Thus, the whole blood that may include an immunosuppressive agent may be whole blood that includes an immunosuppressive agent or whole blood that does not include an immunosuppressive agent. The above method is useful for measuring counts for each whole blood. Therefore, the expression "method for measuring an immunosuppressive agent in whole blood" used in the present invention includes not only determining the concentration of the immunosuppressive agent contained in whole blood but also determining the presence or absence of the immunosuppressive agent in whole blood.

### EXAMPLES

Hereinafter, the present invention will be described with reference to Examples. The following Examples are not intended to limit the invention.

### <Materials used in Test Examples 1 to 4>

Measuring instrument: Lumipulse (registered trademark) L2400 (Fujirebio Inc.)
Measurement reagent:
   Buffer solution containing 0.04% of anti-cyclosporine monoclonal antibody (mouse)-bound ferrite particles (particle solution)
   Buffer solution containing 1 µg/mL of ALP-labeled anti-cyclosporine immune complex monoclonal antibody (alpaca) (labeled body fluid)
   Reagents for Lumipulse Presto (substrate solution and washing solution)

### (1) Test Example 1

### (1-1) Treatment solution and measurement object used in Test Example 1

Treatment solutions used in Test Example 1 were as follows.
Treatment solution 1-A: 100 mM MES (component (C)), 4 M Urea (component (B-1)), 6.0% CHAPS (component (B-2)), 0.75% MEGA8 (component (B-2)), 0.1% ProClin300, 0.005% Antifoam SI, pH 6.5
Treatment solution 1-B: treatment solution 1-A adjusted to a pH of 6.0
Treatment solution 1-C: treatment solution 1-A with 2.2% Brij (registered trademark) 58 (component (A)) added
Treatment solution 1-D: treatment solution 1-A with 0.338% sodium taurocholate added
Treatment solution 1-E: treatment solution 1-B with 2.2% Brij 58 and 0.338% sodium taurocholate added

Measurement objects used in Test Example 1 were as follows.

Measurement object: Phosphate buffer containing cyclosporine (standard solution), fresh whole blood sample not containing cyclosporine (EDTA2Na) (cyclosporine-free specimen, day 4 after blood collection, storage temperature 2 to 8°C)

### (1-2) Experimental Method

The following reactions and measurements were performed with Lumipulse L2400. That is, the measurement object was treated with the treatment solution, and then an immunoassay was performed (each dilution series of labeled body fluid: n=2, each cyclosporine-free specimen (samples 1 to 9): n=2).
(1) To 100 µL of the treatment solution, 20 µL of a measurement object (standard solution and cyclosporine-free specimen) was dispensed, and the mixture was stirred and then incubated at 37°C for 6.5 minutes. For the standard solution, a phosphate buffer was added, and cyclosporine concentrations were diluted to 30, 100, 300, 800, and 2000 ng/mL, and 20 µL each of the diluted solutions was used as a measurement object to prepare a dilution series (Cal 0 to 2000).
(2) 50 µL of the particle solution was dispensed into the reaction solution of (1), and the mixture was stirred and then incubated at 37°C for 8 minutes.
(3) The reaction solution was removed by B/F separation using a magnet, and then the antibody-binding particles were washed with a washing solution.
(4) 50 µL of the labeled body fluid and the antibody-binding particles washed in (3) were mixed and incubated at 37°C for 8 minutes.
(5) The reaction solution was removed by B/F separation using a magnet, and then the antibody-binding particles were washed with a washing solution.
(6) 200 µL of the substrate solution was added to the antibody-bound particles washed in (5) and reacted at 37°C for 4 minutes.
(7) The luminescence intensity (count) at a wavelength of 463 nm was measured. The luminescence intensity was measured using Lumipulse L2400. The measurement results of the luminescence intensity are shown in Table 1.

The method for calculating the measured value of cyclosporine (ng/mL) in a whole blood specimen is as follows.

A calibration curve was drawn using a standard solution, and the measured value of the cyclosporine-free specimen was calculated. More specifically, a linear approximate equation of a calibration curve was obtained from the counts at the time of measuring the luminescence intensity of the standard solution obtained in the above procedures (1) to (7) and the cyclosporine concentration set in the dilution series in the procedure (1). Using a linear approximation equation of a calibration curve, a cyclosporine measured value (ng/mL) was calculated based on the count value at the time of measuring a specimen containing no cyclosporine. The results are shown in Table 2.

The non-specific reaction was evaluated as follows.

It was defined that a non-specific reaction occurred when the measured value of the whole blood specimen reached 30 ng/mL or more, and the occurrence of the non-specific reaction was evaluated. More specifically, the ratio of samples in which the measured value of the whole blood specimen was 30 ng/mL or more in the measurement sample was calculated as the frequency of non-specific reaction. The results are shown in Table 2.

**[Table 1]**

| | | Comparative Example 1 | Example 1 | Example 2 | Comparative Example 2 | Example 3 |
|---|---|---|---|---|---|---|
| Treatment solution | | 1-A | 1-B | 1-C | 1-D | 1-E |
| | pH | 6.5 | 6.0 | 6.5 | 6.5 | 6.0 |
| | Brij 58 (%) | 0 | 0 | 2.2 | 0 | 2.2 |
| | Sodium taurocholate | 0 | 0 | 0 | 0.338 | 0.338 |
| Count | C al 0 | 6,041 | 5,783 | 5,807 | 5,721 | 5,673 |
| | | 5,753 | 6,059 | 6,059 | 5,772 | 5,629 |
| | Cal 30 | 11,017 | 10,918 | 7,938 | 11,141 | 7,683 |
| | | 11,146 | 10,792 | 7,985 | 11,110 | 7,492 |
| | Cal 100 | 43,024 | 38,257 | 16,149 | 40,103 | 13,701 |
| | | 44,317 | 36,025 | 16,734 | 43,845 | 14,566 |
| | Cal 300 | 475,862 | 377,769 | 86,685 | 401,935 | 62,291 |
| | | 471,037 | 364,844 | 86,288 | 403,871 | 60,067 |
| | Cal 800 | 5,610,121 | 4,365,946 | 921,607 | 4,835,610 | 558,818 |
| | | 5,584,699 | 4,377,915 | 898,733 | 4,919,925 | 537,669 |
| | Cal 2000 | 27,457,750 | 22,239,970 | 8,234,899 | 25,505,584 | 5,411,726 |
| | | 26,775,393 | 23,138,517 | 8,151,856 | 25,214,719 | 5,468,395 |
| | Sample 1 | 5,795 | 5,789 | 5,728 | 14,127 | 5,664 |
| | | 19,605 | 5,933 | 5,757 | 24,208 | 6,013 |
| | Sample 2 | 12,152 | 6,055 | 5,692 | 12,189 | 5,999 |
| | | 5,736 | 6,153 | 5,636 | 16,623 | 5,816 |
| | Sample 3 | 8,439 | 6,215 | 6,110 | 12,870 | 5,959 |
| | | 13,853 | 5,953 | 6,102 | 21,236 | 6,006 |
| | Sample 4 | 10,862 | 6,023 | 5,878 | 11,729 | 5,969 |
| | | 5,917 | 6,290 | 5,926 | 8,876 | 6,087 |
| | Sample 5 | 5,662 | 5,910 | 6,558 | 6,350 | 6,001 |
| | | 5,828 | 5,705 | 5,657 | 6,494 | 5,995 |
| | Sample 6 | 8,825 | 6,138 | 5,681 | 6,642 | 6,019 |
| | | 6,033 | 5,953 | 5,809 | 5,863 | 6,338 |
| | Sample 7 | 5,916 | 6,044 | 5,948 | 9,434 | 6,004 |
| | | 5,731 | 6,079 | 6,073 | 8,038 | 6,126 |
| | Sample 8 | 19,657 | 6,091 | 5,990 | 25,054 | 6,088 |
| | | 6,009 | 5,956 | 5,739 | 29,643 | 6,087 |
| | Sample 9 | 9,657 | 5,801 | 5,843 | 6,187 | 5,915 |
| | | 5,815 | 5,979 | 5,902 | 8,323 | 6,120 |

**[Table 2]**

| | | Comparative Example 1 | Example 1 | Example 2 | Comparative Example 2 | Example 3 |
|---|---|---|---|---|---|---|
| Treatment solution | | 1-A | 1-B | 1-C | 1-D | 1-E |
| Measured value (ng/mL) | Sample 1 | 0.0 | 0.0 | 0.0 | 40.9 | 0.2 |
| | | 56.4 | 0.1 | 0.0 | 67.2 | 5.8 |
| | Sample 2 | 34.3 | 0.9 | 0.0 | 34.2 | 5.6 |
| | | 0.0 | 1.6 | 0.0 | 48.5 | 2.7 |
| | Sample 3 | 16.9 | 2.1 | 2.9 | 36.7 | 5.0 |
| | | 40.2 | 0.2 | 2.7 | 60.4 | 5.7 |
| | Sample 4 | 29.1 | 0.7 | 0.0 | 32.4 | 5.1 |
| | | 0.1 | 2.6 | 0.0 | 19.4 | 7.0 |
| | Sample 5 | 0.0 | 0.0 | 10.0 | 4.1 | 5.6 |
| | | 0.0 | 0.0 | 0.0 | 5.0 | 5.5 |
| | Sample 6 | 19.1 | 1.5 | 0.0 | 6.0 | 5.9 |
| | | 1.0 | 0.2 | 0.0 | 0.8 | 11.0 |
| | S ample 7 | 0.1 | 0.9 | 0.2 | 22.3 | 5.7 |
| | | 0.0 | 1.1 | 2.3 | 14.7 | 7.6 |
| | S ample 8 | 56.5 | 1.2 | 0.9 | 69.0 | 7.0 |
| | | 0.8 | 0.2 | 0.0 | 78.3 | 7.0 |
| | S ample 9 | 23.5 | 0.0 | 0.0 | 3.0 | 4.2 |
| | | 0.0 | 0.4 | 0.0 | 16.4 | 7.5 |
| Sensitivity (30/0) * | | 1.88 | 1.83 | 1.34 | 1.94 | 1.34 |
| Frequency of non-specific reaction | | 22% | 0% | 0% | 50% | 0% |

| | | | | | | |
|---|---|---|---|---|---|---|
| [Notes on Table 2] *: "Sensitivity (30/0)" is the ratio of the average value of the luminescence intensity of Cal 30 to the average value of the luminescence intensity of Cal 0 in Table 1. The same applies to Tables 5, 6, and 9 below. | | | | | | |

### (1-3) Results

The non-specific reaction occurred in the treatment solution 1-A, whereas the occurrence of the non-specific reaction was suppressed in the treatment solutions 1-B, 1-C, and 1-E. On the other hand, the treatment solution 1-D using sodium taurocholate instead of Brij 58 could not suppress the non-specific reaction.

### (2) Test Example 2

### (2-1) Treatment solution and measurement object used in Test Example 2

Treatment solutions used in Test Example 2 were as follows.
Treatment solution 2-A: 100 mM MES (component (C)), 4 M Urea (component (B-1)), 6.0% CHAPS (component (B-2)), 0.75% MEGA8 (component (B-2)), 0.1% ProClin300, 0.005% Antifoam SI, pH 6.5
Treatment solution 2-B: treatment solution 2-A adjusted to a pH of 5.8
Treatment solution 2-C: treatment solution 2-A adjusted to a pH of 6.0
Treatment solution 2-D: treatment solution 2-A adjusted to a pH of 6.2
Treatment solution 2-E: treatment solution 2-A with 1.0% Pluronic (registered trademark) F-68 (component (A)) added
Treatment solution 2-F: treatment solution 2-A with 1.0% Pluronic F-108 (component (A)) added

Measurement objects used in Test Example 2 were as follows.

Measurement object: Phosphate buffer solution containing cyclosporine (standard solution), fresh whole blood specimen not containing cyclosporine (EDTA2Na) (cyclosporine-free specimen, day 5 after blood collection, storage temperature 2 to 8°C), specimen obtained by freezing the whole blood specimen in a freezer at -80°C for 2 hours and then thawing the frozen whole blood specimen in a water bath at room temperature (cyclosporine-free frozen specimen)

### (2-2) Experimental method

The luminescence intensity was measured in the same manner as in the procedure of Test Example 1 except that the above-described treatment solutions 2-A to 2-F were used as the treatment solutions and a frozen specimen containing no cyclosporine was also used as the measurement object in addition to the above-described standard solution and the specimen containing no cyclosporine (each dilution series of standard solution: n=3, each cyclosporine-free specimen (sample 10 to 19): n=2, each cyclosporine-free frozen specimen (frozen samples 10 to 19): n=1). The results are shown in Tables 3 and 4.

In addition, in the same manner as in Test Example 1, the cyclosporine measured value (ng/mL) was calculated based on the count values at the time of measurement of the cyclosporine-free specimen and the cyclosporine-free frozen specimen, using a linear approximation equation of a calibration curve obtained from the count values at the time of measurement of the standard solution. The results are shown in Table 5.

In the same manner as in Test Example 1, the sensitivity (30/0) and the frequency of non-specific reactions in the cyclosporine-free specimen and the cyclosporine-free frozen specimen were calculated. The results are shown in Table 5.

**[Table 3]**

| | | Comparative Example 3 | Example 4 | Example 5 | Example 6 | Example 7 | Example 8 |
|---|---|---|---|---|---|---|---|
| Treatment solution | | 2-A | 2-B | 2-C | 2-D | 2-E | 2-F |
| | pH | 6.5 | 5.8 | 6.0 | 6.2 | 6.5 | 6.5 |
| | Pluronic F-68 (%) | 0 | 0 | 0 | 0 | 1 | 0 |
| | Pluronic F-108 (%) | 0 | 0 | 0 | 0 | 0 | 1 |
| Count | Cal 0 | 6,084 | 5,876 | 6,136 | 6,071 | 6,244 | 5,921 |
| | | 5,770 | 6,019 | 6,220 | 6,115 | 5,984 | 5,799 |
| | | 5,972 | 6,023 | 5,893 | 6,192 | 5,840 | 5,836 |
| | Cal 30 | 10,818 | 10,523 | 10,790 | 11,209 | 11,202 | 10,155 |
| | | 11,449 | 10,712 | 10,947 | 10,746 | 11,475 | 10,130 |
| | | 11,741 | 10,633 | 10,425 | 10,930 | 11,413 | 10,229 |
| | Cal 100 | 45,927 | 35,241 | 38,180 | 40,701 | 41,291 | 31,773 |
| | | 44,783 | 35,311 | 38,704 | 40,738 | 40,779 | 31,693 |
| | | 45,870 | 38,002 | 37,958 | 42,583 | 41,150 | 30,602 |
| | Cal 300 | 477,615 | 354,056 | 372,918 | 423,063 | 429,978 | 281,176 |
| | | 491,667 | 331,315 | 391,924 | 434,952 | 418,637 | 271,586 |
| | | 507,501 | 367,186 | 386,237 | 417,682 | 429,591 | 266,544 |
| | Cal 800 | 5,934,352 | 4,159,925 | 4,263,836 | 5,092,579 | 5,229,538 | 3,188,926 |
| | | 5,774,090 | 3,985,257 | 4,558,832 | 5,033,792 | 5,202,528 | 3,253,751 |
| | | 5,827,051 | 4,052,095 | 4,495,172 | 4,993,753 | 5,019,704 | 3,211,276 |
| | Cal 2000 | 27,586,659 | 23,066,383 | 23,550,770 | 24,759,037 | 26,512,385 | 19,546,235 |
| | | 26,132,438 | 23,132,797 | 24,162,371 | 25,192,475 | 24,945,144 | 19,511,492 |
| | | 27,516,008 | 22,866,612 | 24,396,674 | 25,987,323 | 25,141,102 | 19,181,435 |

**[Table 4]**

| | | Comparative Example 3 | Example 4 | Example 5 | Example 6 | Example 7 | Example 8 |
|---|---|---|---|---|---|---|---|
| Treatment solution | | 2-A | 2-B | 2-C | 2-D | 2-E | 2-F |
| | pH | 6.5 | 5.8 | 6.0 | 6.2 | 6.5 | 6.5 |
| | Pluronic F-68 (%) | 0 | 0 | 0 | 0 | 1 | 0 |
| | Pluronic F-108 (%) | 0 | 0 | 0 | 0 | 0 | 1 |
| Count | Sample 10 | 13,766 | 6,046 | 6,104 | 6,115 | 5,829 | 5,955 |
| | | 14,353 | 6,339 | 5,845 | 6,253 | 6,020 | 6,111 |
| | Sample 11 | 14,886 | 6,034 | 6,035 | 6,073 | 5,948 | 5,884 |
| | | 5,876 | 6,115 | 6,392 | 6,035 | 6,134 | 6,029 |
| | Sample 12 | 6,524 | 6,073 | 5,925 | 6,106 | 6,061 | 6,004 |
| | | 9,087 | 5,926 | 6,041 | 5,888 | 6,071 | 5,956 |
| | Sample 13 | 6,326 | 6,329 | 5,898 | 6,049 | 6,341 | 5,807 |
| | | 17,420 | 6,113 | 6,264 | 6,017 | 6,216 | 6,074 |
| | Sample 14 | 6,276 | 6,044 | 6,125 | 6,004 | 6,066 | 5,776 |
| | | 10,897 | 6,103 | 6,203 | 6,249 | 6,080 | 5,862 |
| | Sample 15 | 6,215 | 6,108 | 6,118 | 6,191 | 6,326 | 6,029 |
| | | 6,198 | 6,238 | 5,976 | 5,979 | 6,172 | 5,985 |
| | Sample 16 | 6,297 | 6,077 | 6,132 | 6,006 | 6,378 | 6,234 |
| | | 6,041 | 6,205 | 5,987 | 6,044 | 6,135 | 5,960 |
| | Sample 17 | 6,316 | 6,249 | 6,031 | 5,812 | 6,351 | 6,178 |
| | | 5,960 | 5,876 | 6,052 | 5,744 | 6,086 | 5,998 |
| | Sample 18 | 9,260 | 6,090 | 6,034 | 6,019 | 5,840 | 5,805 |
| | | 6,142 | 6,200 | 6,065 | 5,932 | 6,349 | 5,893 |
| | Sample 19 | 6,226 | 6,308 | 5,849 | 5,814 | 5,788 | 5,877 |
| | | 5,743 | 6,015 | 6,109 | 5,992 | 5,654 | 5,798 |
| | Frozen sample 10 | 5,702 | 6,130 | 5,607 | 5,828 | 5,825 | 5,779 |
| | Frozen sample 11 | 5,912 | 5,786 | 5,927 | 5,960 | 5,837 | 5,910 |
| | Frozen sample 12 | 6,124 | 5,910 | 5,849 | 6,058 | 5,749 | 5,835 |
| | Frozen sample 13 | 6,010 | 6,223 | 6,017 | 6,204 | 6,080 | 5,650 |
| | Frozen sample 14 | 5,892 | 6,270 | 5,981 | 6,071 | 5,910 | 6,030 |
| | Frozen sample 15 | 6,092 | 5,956 | 6,459 | 6,269 | 6,319 | 6,062 |
| | Frozen sample 16 | 6,268 | 5,889 | 5,941 | 6,314 | 6,084 | 6,125 |
| | Frozen sample 17 | 5,934 | 5,949 | 6,055 | 6,020 | 6,253 | 5,876 |
| | Frozen sample 18 | 6,053 | 6,189 | 5,845 | 5,953 | 6,106 | 5,932 |
| | Frozen sample 19 | 5,823 | 6,228 | 6,102 | 5,828 | 5,972 | 5,758 |

**[Table 5]**

| | | Comparative Example 3 | Example 4 | Example 5 | Example 6 | Example 7 | Example 8 |
|---|---|---|---|---|---|---|---|
| Treatment solution | | 2-A | 2-B | 2-C | 2-D | 2-E | 2-F |
| | pH | 6.5 | 5.8 | 6.0 | 6.2 | 6.5 | 6.5 |
| | Pluronic F-68 (%) | 0 | 0 | 0 | 0 | 1 | 0 |
| | Pluronic F-108 (%) | 0 | 0 | 0 | 0 | 0 | 1 |
| Measured value (ng/mL) | Sample 10 | 38.7 | 0.5 | 0.2 | 0.0 | 0.0 | 0.8 |
| | | 40.5 | 2.7 | 0.0 | 1.0 | 0.0 | 2.0 |
| | Sample 11 | 42.1 | 0.5 | 0.0 | 0.0 | 0.0 | 0.3 |
| | | 0.0 | 1.1 | 2.4 | 0.0 | 0.7 | 1.4 |
| | Sample 12 | 4.0 | 0.8 | 0.0 | 0.0 | 0.3 | 1.2 |
| | | 19.7 | 0.0 | 0.0 | 0.0 | 0.3 | 0.8 |
| | Sample 13 | 2.6 | 2.7 | 0.0 | 0.0 | 2.1 | 0.0 |
| | | 49.2 | 1.1 | 1.4 | 0.0 | 1.3 | 1.7 |
| | Sample 14 | 2.3 | 0.5 | 0.3 | 0.0 | 0.3 | 0.0 |
| | | 28.2 | 1.0 | 0.9 | 0.9 | 0.4 | 0.1 |
| | Sample 15 | 1.9 | 1.0 | 0.3 | 0.5 | 2.0 | 1.4 |
| | | 1.8 | 2.0 | 0.0 | 0.0 | 1.0 | 1.0 |
| | Sample 16 | 2.5 | 0.8 | 0.4 | 0.0 | 2.4 | 3.0 |
| | | 0.7 | 1.7 | 0.0 | 0.0 | 0.7 | 0.9 |
| | Sample 17 | 2.6 | 2.1 | 0.0 | 0.0 | 2.2 | 2.6 |
| | | 0.1 | 0.0 | 0.0 | 0.0 | 0.4 | 1.1 |
| | Sample 18 | 20.6 | 0.9 | 0.0 | 0.0 | 0.0 | 0.0 |
| | | 1.4 | 1.7 | 0.0 | 0.0 | 2.2 | 0.3 |
| | Sample 19 | 2.0 | 2.5 | 0.0 | 0.0 | 0.0 | 0.2 |
| | | 0.0 | 0.3 | 0.2 | 0.0 | 0.0 | 0.0 |
| | Frozen sample 10 | 0.0 | 1.2 | 0.0 | 0.0 | 0.0 | 0.0 |
| | Frozen sample 11 | 0.0 | 0.0 | 0.0 | 0.0 | 0.0 | 0.5 |
| | Frozen sample 12 | 1.3 | 0.0 | 0.0 | 0.0 | 0.0 | 0.0 |
| | Frozen sample 13 | 0.5 | 1.9 | 0.0 | 0.6 | 0.4 | 0.0 |
| | Frozen sample 14 | 0.0 | 2.2 | 0.0 | 0.0 | 0.0 | 1.4 |
| | Frozen sample 15 | 1.0 | 0.0 | 2.9 | 1.1 | 2.0 | 1.7 |
| | Frozen sample 16 | 2.3 | 0.0 | 0.0 | 1.4 | 0.4 | 2.1 |
| | Frozen sample 17 | 0.0 | 0.0 | 0.0 | 0.0 | 1.5 | 0.2 |
| | Frozen sample 18 | 0.8 | 1.6 | 0.0 | 0.0 | 0.6 | 0.6 |
| | Frozen sample 19 | 0.0 | 1.9 | 0.1 | 0.0 | 0.0 | 0.0 |
| Sensitivity (30/0) | | 1.91 | 1.78 | 1.76 | 1.79 | 1.89 | 1.74 |
| Frequency of non-specific reaction (Unfrozen specimen) | | 20% | 0% | 0% | 0% | 0% | 0% |
| Frequency of non-specific reaction (Frozen specimen) | | 0% | 0% | 0% | 0% | 0% | 0% |

### (2-3) Results

A non-specific reaction occurred in the treatment solution 2-A having a pH of 6.5 and containing no nonionic surfactant having a polyoxyethylene alcohol structure, whereas no non-specific reaction occurred in any of the treatment solutions 2-B to 2-D having pH of 5.8, 6.0, and 6.2, respectively. In addition, no non-specific reaction occurred in the treatment solutions 2-E and 2-F using Pluronic F-68 or F-108, which is a nonionic surfactant having a polyoxyethylene alcohol structure. In addition, for 10 frozen specimens containing no cyclosporine, no non-specific reaction occurred under any conditions.

### (3) Test Example 3

### (3-1) Treatment solution and measurement object used in Test Example 3

Treatment solutions used in Test Example 3 were as follows.
Treatment solution 3-A: 100 mM MES (component (C)), 4 M Urea (component (B-1)), 6.0% CHAPS (component (B-2)), 0.75% MEGA8 (component (B-2)), 0.1% ProClin300, 0.005% Antifoam SI, pH 6.5
Treatment solution 3-B: treatment solution 3-A adjusted to a pH of 6.0
Treatment solution 3-C: treatment solution 3-A with 1% Pluronic F-68 (component (A)) added
Treatment solution 3-D: treatment solution 3-B with 1% Pluronic F-68 (component (A)) added

Measurement objects used in Test Example 3 were as follows.

Measurement object : Phosphate buffer containing cyclosporine (standard solution), fresh whole blood sample not containing cyclosporine (EDTA2Na) (cyclosporine-free specimen, within 24 hours after blood collection, storage temperature 2 to 8°C)

### (3-2) Experimental method

The luminescence intensity was measured in the same manner as in the procedure of Test Example 1 except that the above-described treatment solutions 3-A to 3-D were used as the treatment solutions (Each dilution series of standard solution: n=2, each cyclosporine-free specimen (samples 20 to 58): n=1). The results are shown in 6 and 7.

In addition, in the same manner as in Test Example 1, a cyclosporine measured value (ng/mL) was calculated based on the count of the specimen not containing cyclosporine, using a linear approximation equation of a calibration curve obtained from the count at the time of measuring the standard solution. The results are shown in Table 8.

In the same manner as in Test Example 1, the sensitivity (30/0) and the frequency of non-specific reactions in the cyclosporine-free specimens were calculated. The results of the sensitivity (30/0) are shown in Table 6, and the results of the frequency of non-specific reactions are shown in Table 8.

**[Table 6]**

| | | Comparative Example 4 | Example 9 | Example 10 | Example 11 |
|---|---|---|---|---|---|
| Treatment solution | Composition | 3-A | 3-B | 3-C | 3-D |
| | pH | 6.5 | 6.0 | 6.5 | 6.0 |
| | Pluronic F-68 (%) | 0 | 0 | 1 | 1 |
| Count | Cal0 | 7,493 | 8,024 | 7,762 | 7,746 |
| | | 7,950 | 7,714 | 8,197 | 7,525 |
| | Cal30 | 16,221 | 14,447 | 15,469 | 13,910 |
| | | 15,661 | 14,356 | 14,715 | 15,350 |
| | Cal100 | 68,961 | 53,642 | 61,227 | 55,836 |
| | | 68,345 | 54,576 | 59,339 | 48,663 |
| | Cal300 | 784,129 | 531,299 | 644,178 | 482,216 |
| | | 675,050 | 531,928 | 628,613 | 494,903 |
| | Cal800 | 8,518,378 | 6,386,063 | 6,736,734 | 5,099,620 |
| | | 6,704,897 | 5,627,345 | 6,590,259 | 5,351,114 |
| | Cal2000 | 31,639,312 | 26,249,947 | 28,710,028 | 26,764,387 |
| | | 28,777,712 | 25,670,115 | 29,124,375 | 25,398,007 |
| Sensitivity (30/0) | | 2.06 | 1.83 | 1.89 | 1.92 |

**[Table 7]**

| | | Comparative Example 4 | Example 9 | Example 10 | Example 11 |
|---|---|---|---|---|---|
| Treatment solution | Composition | 3-A | 3-B | 3-C | 3-D |
| | pH | 6.5 | 6.0 | 6.5 | 6.0 |
| | Pluronic F-68 (%) | 0 | 0 | 1 | 1 |
| Count | Sample 20 | 28,484 | 7,857 | 8,235 | 8,056 |
| | Sample 21 | 27,993 | 7,927 | 8,113 | 8,216 |
| | Sample 22 | 10,459 | 8,641 | 7,859 | 7,733 |
| | Sample 23 | 8,369 | 8,070 | 8,841 | 8,151 |
| | Sample 24 | 22,499 | 8,057 | 7,821 | 7,768 |
| | Sample 25 | 23,796 | 8,000 | 7,747 | 8,294 |
| | Sample 26 | 13,698 | 8,015 | 7,999 | 8,138 |
| | Sample 27 | 16,860 | 7,897 | 8,352 | 8,653 |
| | Sample 28 | 29,578 | 7,839 | 8,207 | 8,360 |
| | Sample 29 | 28,985 | 8,506 | 8,018 | 8,215 |
| | Sample 30 | 26,980 | 8,249 | 8,160 | 7,937 |
| | Sample 31 | 32,028 | 7,882 | 7,868 | 7,765 |
| | Sample 32 | 23,570 | 8,395 | 8,110 | 8,522 |
| | Sample 33 | 20,337 | 7,775 | 7,859 | 7,976 |
| | Sample 34 | 32,445 | 8,512 | 8,106 | 8,041 |
| | Sample 35 | 28,549 | 8,204 | 7,939 | 7,905 |
| | Sample 36 | 27,336 | 8,124 | 8,377 | 8,473 |
| | Sample 37 | 30,319 | 8,361 | 8,053 | 7,900 |
| | Sample 38 | 29,929 | 7,325 | 7,342 | 7,598 |
| | Sample 39 | 29,175 | 8,004 | 8,354 | 7,951 |
| | Sample 40 | 22,775 | 8,046 | 7,595 | 7,895 |
| | Sample 41 | 52,536 | 8,064 | 8,120 | 8,084 |
| | Sample 42 | 26,823 | 7,790 | 8,129 | 8,068 |
| | Sample 43 | 17,794 | 7,847 | 7,723 | 7,790 |
| | Sample 44 | 22,700 | 7,709 | 8,099 | 7,682 |
| | Sample 45 | 26,758 | 8,119 | 8,040 | 7,759 |
| | Sample 46 | 25,326 | 8,057 | 8,355 | 7,848 |
| | Sample 47 | 15,944 | 8,195 | 7,964 | 8,112 |
| | Sample 48 | 35,378 | 8,741 | 8,415 | 8,673 |
| | Sample 49 | 31,684 | 8,031 | 8,319 | 7,970 |
| | Sample 50 | 35,676 | 8,287 | 8,467 | 8,051 |
| | Sample 51 | 29,130 | 8,239 | 8,614 | 8,286 |
| | Sample 52 | 28,005 | 8,110 | 8,440 | 8,188 |
| | Sample 53 | 27,248 | 8,376 | 8,691 | 8,414 |
| | Sample 54 | 52,844 | 8,317 | 8,528 | 8,102 |
| | Sample 55 | 27,273 | 8,596 | 8,280 | 8,381 |
| | Sample 56 | 26,714 | 8,226 | 8,527 | 8,276 |
| | Sample 57 | 78,614 | 8,172 | 9,145 | 8,832 |
| | Sample 58 | 35,578 | 8,215 | 8,096 | 8,034 |

**[Table 8]**

| | | Comparative Example 4 | Example 9 | Example 10 | Example 11 |
|---|---|---|---|---|---|
| Treatment solution | Composition | 3-A | 3-B | 3-C | 3-D |
| | pH | 6.5 | 6.0 | 6.5 | 6.0 |
| | Pluronic F-68 (%) | 0 | 0 | 1 | 1 |
| Measured value (ng/mL) | Sample 20 | 54.7 | 0.0 | 1.3 | 2.1 |
| | Sample 21 | 53.9 | 0.3 | 0.7 | 2.9 |
| | Sample 22 | 11.9 | 4.3 | 0.0 | 0.5 |
| | Sample 23 | 2.9 | 1.1 | 4.4 | 2.6 |
| | Sample 24 | 44.5 | 1.1 | 0.0 | 0.7 |
| | Sample 25 | 46.9 | 0.7 | 0.0 | 3.3 |
| | Sample 26 | 23.5 | 0.8 | 0.1 | 2.5 |
| | Sample 27 | 32.4 | 0.2 | 1.9 | 5.0 |
| | Sample 28 | 56.4 | 0.0 | 1.2 | 3.6 |
| | Sample 29 | 55.5 | 3.6 | 0.2 | 2.9 |
| | Sample 30 | 52.3 | 2.1 | 0.9 | 1.5 |
| | Sample 31 | 60.0 | 0.1 | 0.0 | 0.6 |
| | Sample 32 | 46.4 | 3.0 | 0.7 | 4.4 |
| | Sample 33 | 40.2 | 0.0 | 0.0 | 1.7 |
| | Sample 34 | 60.6 | 3.6 | 0.6 | 2.0 |
| | Sample 35 | 54.8 | 1.9 | 0.0 | 1.3 |
| | Sample 36 | 52.9 | 1.4 | 2.0 | 4.2 |
| | Sample 37 | 57.5 | 2.8 | 0.4 | 1.3 |
| | Sample 38 | 56.9 | 0.0 | 0.0 | 0.0 |
| | Sample 39 | 55.8 | 0.8 | 1.9 | 1.6 |
| | Sample 40 | 45.0 | 1.0 | 0.0 | 1.3 |
| | Sample 41 | 84.7 | 1.1 | 0.7 | 2.2 |
| | Sample 42 | 52.0 | 0.0 | 0.8 | 2.2 |
| | Sample 43 | 34.6 | 0.0 | 0.0 | 0.8 |
| | Sample 44 | 44.8 | 0.0 | 0.6 | 0.2 |
| | Sample 45 | 51.9 | 1.4 | 0.3 | 0.6 |
| | Sample 46 | 49.5 | 1.1 | 1.9 | 1.1 |
| | Sample 47 | 30.0 | 1.8 | 0.0 | 2.4 |
| | Sample 48 | 64.7 | 4.9 | 2.2 | 5.1 |
| | Sample 49 | 59.5 | 0.9 | 1.7 | 1.7 |
| | Sample 50 | 65.1 | 2.4 | 2.5 | 2.1 |
| | Sample 51 | 55.7 | 2.1 | 3.3 | 3.2 |
| | Sample 52 | 54.0 | 1.4 | 2.4 | 2.7 |
| | Sample 53 | 52.7 | 2.9 | 3.6 | 3.9 |
| | Sample 54 | 85.0 | 2.5 | 2.8 | 2.3 |
| | Sample 55 | 52.8 | 4.1 | 1.5 | 3.7 |
| | Sample 56 | 51.9 | 2.0 | 2.8 | 3.2 |
| | Sample 57 | 108.4 | 1.7 | 5.9 | 5.9 |
| | Sample 58 | 64.9 | 2.0 | 0.6 | 2.0 |
| Frequency of non-specific reaction | | 92% | 0% | 0% | 0% |

### (3-3) Results

The non-specific reaction occurred in the treatment solution 3-A having a pH of 6.5 and containing no surfactant having a polyoxyethylene alcohol structure, whereas the occurrence of the non-specific reaction was suppressed in the treatment solutions 3-B to 3-D.

### (4) Test Example 4

### (4-1) Treatment solution and measurement object used in Test Example 4

Materials used in Test Example 4 were as follows.
Treatment solution 4-A: 100 mM MES (component (C)), 4 M Urea (component (B-1)), 6.0% CHAPS (component (B-2)), 0.75% MEGA8 (component (B-2)), 0.1% ProClin300, 0.005% Antifoam SI, pH 6.5
Treatment solutions 4-B to 4-F: treatment solution 4-A with 1%, 0.5%, 1.4%, 2%, and 3% Pluronic F-68 (component (A)) added, respectively
Treatment solutions 4-G and 4-H: treatment solution 4-A adjusted to a pH of 5.0 and 5.5, respectively

Measurement objects used in Test Example 4 were as follows.

Measurement object: Phosphate buffer containing cyclosporine (standard solution), fresh whole blood sample not containing cyclosporine (EDTA2Na) (cyclosporine-free specimen, day 3 after blood collection, storage temperature 2 to 8°C)

### (4-2) Experimental method

The luminescence intensity was measured in the same manner as in the procedure of Test Example 1 except that the above-described treatment solutions 4-A to 4-H were used as the treatment solutions (each dilution series of standard solution: n=2, each specimen (samples 59 to 75): n=1). The results are shown in Tables 9 and 10.

In the same manner as in Test Example 1, the measured value of cyclosporine (ng/mL) was calculated based on the count value at the time of measuring a specimen containing no cyclosporine, using a linear approximation formula of a calibration curve obtained from the count value at the time of measuring a standard solution. The results are shown in Table 11.

In the same manner as in Test Example 1, the sensitivity (30/0) and the frequency of non-specific reactions in the cyclosporine-free specimens were calculated. The results of the sensitivity (30/0) are shown in Table 9, and the results of the frequency of non-specific reactions are shown in Table 11.

**[Table 9]**

| | | Comparative Example 5 | Example 12 | Example 13 | Example 14 |
|---|---|---|---|---|---|
| Treatment solution | Composition | 4-A | 4-B | 4-C | 4-D |
| | Pluronic F-68 | - | 1.0% | 0.5% | 1.4% |
| | pH | pH 6.5 | pH 6.5 | pH 6.5 | pH 6.5 |
| Count average | Cal 0 | 7,610 | 7,400 | 7,383 | 7,630 |
| | Cal 30 | 14,837 | 14,207 | 14,381 | 13,928 |
| | Cal 100 | 56,007 | 53,790 | 55,350 | 51,372 |
| | Cal 300 | 603,990 | 575,695 | 585,545 | 522,478 |
| | Cal 800 | 7,102,721 | 6,472,332 | 6,805,314 | 6,061,137 |
| | Cal2000 | 32,835,832 | 31,790,138 | 32,300,679 | 30,864,186 |
| Sensitivity (30/0) | | 1.95 | 1.92 | 1.95 | 1.83 |

| | | Example 15 | Example 16 | Example 17 | Example 18 |
|---|---|---|---|---|---|
| Treatment solution | Composition | 4-E | 4-F | 4-G | 4-H |
| | Pluronic F-68 | 2.0% | 3.0% | - | - |
| | pH | pH 6.5 | pH 6.5 | pH 5 | pH 5.5 |
| Count average | Cal 0 | 7,562 | 7,606 | 7,596 | 7,816 |
| | Cal 30 | 14,014 | 13,495 | 11,812 | 12,731 |
| | Cal 100 | 48,617 | 44,723 | 30,670 | 37,455 |
| | Cal 300 | 491,242 | 434,438 | 223,683 | 305,978 |
| | Cal 800 | 5,639,033 | 5,039,423 | 2,498,578 | 3,690,946 |
| | Cal 2000 | 29,037,054 | 27,321,285 | 18,207,708 | 22,890,169 |
| Sensitivity (30/0) | | 1.85 | 1.77 | 1.56 | 1.63 |

**[Table 10]**

| | | Comparative Example 5 | Example 12 | Example 13 | Example 14 | Example 15 | Example 16 | Example 17 | Example 18 |
|---|---|---|---|---|---|---|---|---|---|
| Composition | | 4-A | 4-B | 4-C | 4-D | 4-E | 4-F | 4-G | 4-H |
| Treatment solution | Pluronic F-68 pH | - | 1.0% | 0.5% | 1.4% | 2.0% | 3.0% | - | - |
| | | pH 6.5 | pH 6.5 | pH 6.5 | pH 6.5 | pH 6.5 | pH 6.5 | pH 5 | pH 5.5 |
| Count | Sample 59 | 7,642 | 7,228 | 7,327 | 7,403 | 7,109 | 7,252 | 7,384 | 7,368 |
| | Sample 60 | 7,559 | 7,306 | 7,087 | 7,578 | 7,368 | 7,355 | 7,712 | 7,561 |
| | Sample 61 | 7,567 | 7,404 | 7,387 | 7,499 | 7,165 | 7,302 | 7,872 | 7,740 |
| | Sample 62 | 12,316 | 7,208 | 7,481 | 7,630 | 7,346 | 7,687 | 7,823 | 7,497 |
| | Sample 63 | 7,875 | 7,379 | 7,550 | 7,736 | 7,287 | 7,432 | 8,043 | 7,741 |
| | Sample 64 | 7,322 | 7,561 | 7,600 | 7,405 | 7,376 | 7,425 | 7,748 | 7,657 |
| | Sample 65 | 15,734 | 7,549 | 7,673 | 7,586 | 7,387 | 7,290 | 7,752 | 7,422 |
| | Sample 66 | 7,703 | 7,277 | 7,670 | 7,514 | 7,376 | 7,396 | 7,748 | 7,562 |
| | Sample 67 | 7,618 | 7,384 | 7,264 | 7,404 | 7,310 | 7,317 | 7,673 | 7,649 |
| | Sample 68 | 16,476 | 7,751 | 7,592 | 7,347 | 7,265 | 7,395 | 7,471 | 7,674 |
| | Sample 69 | 7,591 | 7,239 | 7,562 | 7,739 | 7,292 | 7,359 | 7,635 | 7,755 |
| | Sample 70 | 12,435 | 7,022 | 7,386 | 7,706 | 7,403 | 7,337 | 7,948 | 7,851 |
| | Sample 71 | 7,454 | 7,482 | 7,387 | 7,509 | 7,783 | 7,406 | 7,607 | 7,464 |
| | Sample 72 | 7,805 | 7,584 | 7,495 | 7,604 | 7,629 | 7,457 | 7,639 | 7,723 |
| | Sample 73 | 19,677 | 7,042 | 7,279 | 7,023 | 7,508 | 7,454 | 7,605 | 7,726 |
| | Sample 74 | 7,672 | 7,461 | 7,659 | 7,548 | 7,616 | 7,139 | 7,787 | 7,367 |
| | Sample 75 | 7,699 | 7,493 | 7,693 | 7,716 | 7,433 | 7,337 | 7,943 | 7,644 |

**[Table 11]**

| | | Comparative Example 5 | Example 12 | Example 13 | Example 14 | Example 15 | Example 16 | Example 17 | Example 18 |
|---|---|---|---|---|---|---|---|---|---|
| Treatment solution | Composition | 4-A | 4-B | 4-C | 4-D | 4-E | 4-F | 4-G | 4-H |
| | Pluronic F-68 | - | 1.0% | 0.5% | 1.4% | 2.0% | 3.0% | - | - |
| | pH | pH 6.5 | pH 6.5 | pH 6.5 | pH 6.5 | pH 6.5 | pH 6.5 | pH 5 | pH 5.5 |
| Measured value (ng/mL) | Sample 59 | 0.2 | 0.0 | 0.0 | 0.0 | 0.0 | 0.0 | 0.0 | 0.0 |
| | Sample 60 | 0.0 | 0.0 | 0.0 | 0.0 | 0.0 | 0.0 | 0.9 | 0.0 |
| | Sample 61 | 0.0 | 0.0 | 0.0 | 0.0 | 0.0 | 0.0 | 2.2 | 0.0 |
| | Sample 62 | 21.2 | 0.0 | 0.5 | 0.0 | 0.0 | 0.5 | 1.8 | 0.0 |
| | Sample 63 | 1.3 | 0.0 | 0.9 | 0.6 | 0.0 | 0.0 | 3.5 | 0.0 |
| | Sample 64 | 0.0 | 0.8 | 1.1 | 0.0 | 0.0 | 0.0 | 1.2 | 0.0 |
| | Sample 65 | 32.7 | 0.8 | 1.5 | 0.0 | 0.0 | 0.0 | 1.2 | 0.0 |
| | Sample 66 | 0.5 | 0.0 | 1.5 | 0.0 | 0.0 | 0.0 | 1.2 | 0.0 |
| | Sample 67 | 0.0 | 0.0 | 0.0 | 0.0 | 0.0 | 0.0 | 0.6 | 0.0 |
| | Sample 68 | 34.9 | 1.8 | 1.1 | 0.0 | 0.0 | 0.0 | 0.0 | 0.0 |
| | Sample 69 | 0.0 | 0.0 | 0.9 | 0.6 | 0.0 | 0.0 | 0.3 | 0.0 |
| | Sample 70 | 21.6 | 0.0 | 0.0 | 0.4 | 0.0 | 0.0 | 2.8 | 0.2 |
| | Sample 71 | 0.0 | 0.4 | 0.0 | 0.0 | 1.2 | 0.0 | 0.1 | 0.0 |
| | Sample 72 | 1.0 | 1.0 | 0.6 | 0.0 | 0.4 | 0.0 | 0.3 | 0.0 |
| | Sample 73 | 43.2 | 0.0 | 0.0 | 0.0 | 0.0 | 0.0 | 0.1 | 0.0 |
| | Sample 74 | 0.3 | 0.3 | 1.4 | 0.0 | 0.3 | 0.0 | 1.5 | 0.0 |
| | Sample 75 | 0.4 | 0.5 | 1.6 | 0.5 | 0.0 | 0.0 | 2.7 | 0.0 |
| Non-specific reaction Frequency | 18% | 0% | 0% | 0% | 0% | 0% | 0% | 0% | |

### (4-3) Results

The non-specific reaction occurred in the treatment solution 4-A having a pH of 6.5 and containing no POE-based surfactant, whereas the occurrence of the non-specific reaction was suppressed in the treatment solutions 4-B to 4-H.

### (5) Test Example 5

### (5-1) Treatment solution and measurement object used in Test Example 5

Treatment solutions used in Test Example 5 were as follows.
Treatment solution 5-A: 100 mM MES (component (C)), 4M Urea (component (B-1)), 6.0% CHAPS (component (B-2)), 0.75% MEGA8 (component (B-2)), 0.1% ProClin300, 0.005% Antifoam SI, pH 6.5
Treatment solution 5-B: treatment solution 5-A with 1% Pluronic F-68 (component (A)) added
Treatment solution 5-C: treatment solution 4-A adjusted to a pH of 6.0

Measurement objects used in Test Example 5 were as follows.

Measurement object: a phosphate buffer solution containing cyclosporine (standard solution), a specimen obtained by adding cyclosporine to a fresh whole blood specimen not containing cyclosporine (EDTA2Na) (cyclosporine-containing specimen, cyclosporine concentration 50 ng/mL, within 48 hours after blood collection, storage temperature 2 to 8°C), and a specimen obtained by melting (melting in a water bath at room temperature) a purchased whole blood specimen (stored in a freezer at -80°C) and adding cyclosporine (cyclosporine-containing frozen specimen, cyclosporine concentration 50 ng/mL)

### (5-2) Experimental method

The luminescence intensity was measured in the same manner as in the procedure of Test Example 1 except that the above-described treatment solutions 5-A to 5-C were used as treatment solutions, and a cyclosporine-containing specimen and a cyclosporine-containing frozen specimen were used as measurement targets instead of a cyclosporine-free specimen (each dilution series of standard solution: n=2, each cyclosporine-containing specimen (samples 76 to 85): n=2, each cyclosporine-containing frozen specimen (sample 86): n=2). The results of each dilution series of the standard solution are shown in Table 12, and the results of the cyclosporine-containing specimen and the cyclosporine-containing frozen specimen are shown in Table 13.

In addition, in the same manner as in Test Example 1, the cyclosporine measured value (ng/mL) was calculated based on the count values at the time of measurement of the cyclosporine-containing specimen and the cyclosporine-containing frozen specimen, using a linear approximation equation of a calibration curve obtained from the count values at the time of measurement of the standard solution. The results are shown in Table 14.

In the same manner as in Test Example 1, the sensitivity (30/0) and the frequency of non-specific reactions in the cyclosporine-containing specimens were calculated. The results are shown in Table 12.

In Test Example 5, it was defined that a non-specific reaction occurred when the measured value of a specimen (cyclosporine-containing specimen) obtained by adding cyclosporine to a fresh whole blood specimen became 120% or more with respect to the measured value of a specimen (cyclosporine-containing frozen specimen) obtained by adding cyclosporine to a purchased whole blood specimen, and the occurrence of a non-specific reaction was evaluated. The results of the frequency of non-specific reactions are shown in Table 14.

**[Table 12]**

| | | Comparative Example 6 | Example 19 | Example 20 |
|---|---|---|---|---|
| Treatment solution | Composition | 5-A | 5-B | 5-C |
| | Pluronic F-68 | - | 1% | - |
| | pH | pH 6.5 | pH 6.5 | pH 6.0 |
| Count | C al 0 | 7187 | 7280 | 7351 |
| | | 7362 | 7354 | 7402 |
| | C al 30 | 14826 | 14340 | 13812 |
| | | 14578 | 13438 | 13277 |
| | C al 100 | 56890 | 51446 | 47741 |
| | | 57533 | 52144 | 50275 |
| | C al 300 | 642559 | 540173 | 497010 |
| | | 619382 | 526619 | 500507 |
| | C al 800 | 7311986 | 6456381 | 6237755 |
| | | 7480909 | 6493945 | 6055319 |
| | C al 2000 | 34682547 | 31744216 | 30664717 |
| | | 32496063 | 32201737 | 29212672 |
| Sensitivity (30/0) | | 2.02 | 1.90 | 1.84 |

**[Table 13]**

| | | Comparative Example 6 | Example 19 | Example 20 |
|---|---|---|---|---|
| Treatment solution | Composition | 5-A | 5-B | 5-C |
| | Pluronic F-68 | - | 1% | - |
| | pH | pH 6.5 | pH 6.5 | pH 6.0 |
| Count | Sample 76 | 22018 | 19426 | 22138 |
| | | 47897 | 20117 | 24197 |
| | Sample 77 | 23774 | 22495 | 24110 |
| | | 44169 | 22319 | 22933 |
| | Sample 78 | 21665 | 22561 | 23494 |
| | | 48174 | 23075 | 24191 |
| | Sample 79 | 23736 | 22637 | 23154 |
| | | 64700 | 22357 | 24060 |
| | Sample 80 | 42287 | 21447 | 23453 |
| | | 24204 | 21930 | 24184 |
| | Sample 81 | 60605 | 19759 | 23318 |
| | | 24413 | 21785 | 24006 |
| | Sample 82 | 59851 | 22369 | 23673 |
| | | 24539 | 23254 | 25136 |
| | Sample 83 | 52914 | 22026 | 23560 |
| | | 43085 | 21844 | 22760 |
| | Sample 84 | 22427 | 21310 | 23252 |
| | | 58306 | 21993 | N.D. |
| | Sample 85 | 22481 | 22153 | 22559 |
| | | 45571 | 22109 | N.D. |
| | Sample 86 (Frozen whole blood specimen) | 22641 | 20630 | 22243 |
| | | 23414 | 21948 | 22098 |

**[Table 14]**

| | | | Comparative Example 6 | Example 19 | Example 20 |
|---|---|---|---|---|---|
| Measured value (ng/mL) | Cyclosporine-containing specimen | Sample 76 | 48.4 | 45.8 | 54.0 |
| | | | 89.1 | 47.5 | 58.5 |
| | | Sample 77 | 52.1 | 52.9 | 58.3 |
| | | | 84.4 | 52.5 | 55.8 |
| | | Sample 78 | 47.7 | 53.0 | 57.0 |
| | | | 89.4 | 54.1 | 58.5 |
| | | Sample 79 | 52.0 | 53.2 | 56.3 |
| | | | 108.0 | 52.6 | 58.2 |
| | | Sample 80 | 81.9 | 50.6 | 56.9 |
| | | | 52.9 | 51.6 | 58.5 |
| | | Sample 81 | 103.7 | 46.6 | 56.6 |
| | | | 53.3 | 51.3 | 58.1 |
| | | Sample 82 | 102.9 | 52.6 | 57.4 |
| | | | 53.6 | 54.5 | 60.5 |
| | | Sample 83 | 95.1 | 51.9 | 57.1 |
| | | | 82.9 | 51.4 | 55.4 |
| | | Sample 84 | 49.3 | 50.2 | 56.5 |
| | | | 101.2 | 51.8 | N.D. ^{*1} |
| | | Sample 85 | 49.4 | 52.1 | 54.9 |
| | | | 86.2 | 52.0 | N.D. ^{*1} |
| | cyclosporine containing frozen specimen | Sample 86 | 49.8 | 48.7 | 54.2 |
| | | | 51.3 | 51.7 | 53.9 |
| | | Frozen whole blood avg. *² | 50.6 | 50.2 | 54.1 |
| | | Frozen whole blood avg. × 1.2 ^{*3} | 60.7 | 60.2 | 64.9 |
| Frequency of non-specific reaction | | | 55% | 0% | 0% |

| | | | | | |
|---|---|---|---|---|---|
| [Notes on Table 14] *1: "N.D." stands for No Data. *2: "Frozen whole blood avg." is an average value of the measured values n=2 of the cyclosporine-containing frozen specimens (sample 86) treated with each treatment solution. *3: "Frozen whole blood avg. × 1.2" is a numerical value obtained by multiplying "Frozen whole blood avg." by 1.2. That is, in Test Example 5, it was defined that a non-specific reaction occurred when the measured value of the cyclosporine-containing specimen exceeded the value of "Frozen whole blood avg. ×1.2". | | | | | |

### (5-3) Results

The non-specific reaction occurred in the treatment solution 5-A having a pH of 6.5 and containing no surfactant having a polyoxyethylene alcohol structure, whereas the occurrence of the non-specific reaction was suppressed in the treatment solutions B to C.

## Claims

1. A method for measuring an immunosuppressive agent in whole blood, the method comprising:
(a) mixing whole blood possibly containing an immunosuppressive agent with a treatment solution to prepare a mixture solution; and
(b) measuring a concentration of the immunosuppressive agent in the mixture solution by immunoassay,
wherein the treatment solution contains an extractant, and
wherein the treatment solution further contains a nonionic surfactant having a polyoxyethylene alcohol structure at a concentration of 0.5 to 5 mass% and/or has a pH of 4.8 to 6.4.

2. The method according to claim 1, wherein the nonionic surfactant having the polyoxyethylene alcohol structure is at least one selected from the group consisting of an alcohol ethoxylate and a polyoxyethylene-polyoxypropylene block copolymer.

3. The method according to claim 1 or 2, wherein the treatment solution contains the nonionic surfactant having the polyoxyethylene alcohol structure at a concentration of 0.9 to 1.4 mass%.

4. The method according to claim 1 or 2, wherein the treatment solution further contains a pH buffering agent.

5. The method according to claim 4, wherein the pH buffering agent is at least one selected from the group consisting of 2-morpholinoethanesulfonate, phosphate, tartrate, citrate, acetate, trifluoroacetate, phthalate, glycine, and carbonate.

6. The method according to claim 1 or 2, wherein the immunosuppressive agent is a cyclic polypeptide immunosuppressive agent or a macrolide immunosuppressive agent.

7. The method according to claim 1 or 2, wherein the immunosuppressive agent is at least one selected from the group consisting of cyclosporine, tacrolimus, everolimus, sirolimus, temsirolimus, zotarolimus, biolimus, novolimus, pimecrolimus, myolimus, myolimus, and deforolimus.

8. The method according to claim 1 or 2, wherein the extractant contains a chaotropic denaturant and/or a surfactant other than the nonionic surfactant having the polyoxyethylene alcohol structure.

9. The method according to claim 1 or 2, wherein non-specific reaction in the immunoassay is suppressed by the treatment solution which contains the nonionic surfactant having the polyoxyethylene alcohol structure at a concentration of 0.5 to 5 mass% and/or which has a pH of 4.8 to 6.4.

10. The method according to claim 1 or 2, wherein the treatment solution contains a nonionic surfactant having the polyoxyethylene alcohol structure at a concentration of 0.5 to 5 mass% and has a pH of 4.8 to 6.4.

11. A kit for measuring an immunosuppressive agent in whole blood by immunoassay, the kit comprising a treatment solution,
wherein the treatment solution contains an extractant, and
wherein the treatment solution further contains a nonionic surfactant having a polyoxyethylene alcohol structure at a concentration of 0.5 to 5 mass% and/or has a pH of 4.8 to 6.4.

12. A method for suppressing a non-specific reaction in immunoassay for measuring an immunosuppressive agent in whole blood, the method comprising:
(a) mixing whole blood possibly containing an immunosuppressive agent with a treatment solution to prepare a mixture solution,
wherein the treatment solution contains an extractant, and
wherein the treatment solution further contains a nonionic surfactant having a polyoxyethylene alcohol structure at a concentration of 0.5 to 5 mass% and/or has a pH of 4.8 to 6.4.
